(19) **Europäisches Patentamt**

**European Patent Office**

**Office européen des brevets**

(11) **EP 1 665 977 A1**

(12) **EUROPEAN PATENT APPLICATION**
published in accordance with Art. 158(3) EPC

(43) Date of publication:
**07.06.2006 Bulletin 2006/23**

(51) Int Cl.:
***A61B 1/00*** (1968.09)

(21) Application number: **04772976.9**

(22) Date of filing: **07.09.2004**

(86) International application number:
**PCT/JP2004/013294**

(87) International publication number:
**WO 2005/027737 (31.03.2005 Gazette 2005/13)**

(84) Designated Contracting States:
**DE FR GB**

(30) Priority: **18.09.2003 JP 2003326511**

(71) Applicant: **Olympus Corporation**
**Tokyo 151-0072 (JP)**

(72) Inventors:
- **NAKAMURA, Tsutomu**
  **Kamiina-gun, Nagano 399-4511 (JP)**
- **YOSHIZAWA, Fukashi**
  **Ina-shi, Nagano 396-0021 (JP)**

- **MATSUMOTO, Kazuya**
  **Kamiina-gun, Nagano 399-4511 (JP)**
- **SHIMIZU, Hatsuo**
  **Hachioji-shi, Tokyo 192-0024 (JP)**

(74) Representative: **Winter, Brandl, Fürniss, Hübner**
**Röss, Kaiser,**
**Polte Partnerschaft Patent- und**
**Rechtsanwaltskanzlei**
**Alois-Steinecker-Strasse 22**
**D-85354 Freising (DE)**

(54) **ENERGY SUPPLY COIL AND RADIO SYSTEM FOR ACQUIRING INFORMATION IN OBJECT USING IT**

(57)      An object of the present invention is to provide an energy supplying coil that realizes high energy efficiency under low voltage, and a wireless system for obtaining information inside a subject using the energy supplying coil. The energy supplying coil supplies energy to a capsule endoscope which is used in a state of being inserted into the subject to execute a predetermined function inside the subject. The energy supplying coil is wound around an outside of the subject into which the capsule endoscope passes, the capsule endoscope being disposed in an internal space of the wound coil.

FIG.4

**Description**

TECHNICAL FIELD

The present invention relates to an energy supplying coil that supplies energy from the outside of a subject to a body-insertable device used in a state of being introduced into the subject to execute a predetermined function within the subject, and a wireless system for obtaining information inside the subject using the energy supplying coil.

BACKGROUND ART

Swallowing capsule endoscopes have been known in the fields of endoscopes in recent years. The capsule endoscopes have an imaging function and a wireless communication function. While a capsule endoscope is swallowed through a mouth of a patient for observation (examination) and then comes out of a body naturally, the capsule endoscope moves in a body cavity including internal organs such as stomach and small intestine according to their peristaltic movement so as to successively capture their images.

Image data captured in a body by the capsule endoscope, while moving in the body cavity, is successively transmitted to an outside through wireless communication, and is stored in a memory. When a patient carries a receiver having the wireless communication function and the memory function, the patient can move freely even while the patient swallows the capsule endoscope and then the capsule comes out of the body. Thereafter, doctors or nurses make a display device display images of organs based on the image data stored in the memory so as to be capable of making a diagnosis.

While a driving power of such capsule endoscopes may be fed from a built-in power supply, in recent years an attention has been paid to a configuration in which the driving power is fed from the outside via wireless transmission to the capsule endoscopes. Such a configuration in which the power is fed from the outside can avoid whole power from being consumed involuntarily and the driving from being stopped when the capsule endoscope moves in a body cavity.

In the meantime, "A research into a wireless control and energy supply for a micromachine" by OIWA et al, the preparatory paper for the lecture meeting at the Japan Society for Precision Engineering autumn meeting in 1993, pp. 99-101 describes a system that supplies energy to a micromachine using a power feeding coil. This power feeding system supplies a voltage of 520 volts peak-to-peak across terminals of the power feeding coil wound by 50 turns around a 300 millimeter square wooden frame, to send energy of about 34 watts, and get 20 to 30 milliwatt in a power receiving coil. Transmission efficiency is about 0.065 to 0.09 percent.

However, if the power feeding system disclosed in the document is intended to adopt for supplying power to a capsule endoscope, there exists a great disadvantage in energy efficiency, with 0.1 or less percent of transmission efficiency. It is preferable that the subject carry an energy source for supplying power to the capsule endoscope. Therefore, the energy source desirably has as high energy efficiency as possible.

In the power feeding system described in the above document, a high voltage exceeding 500 volts is applied to the power feeding coil. Therefore, there exists a problem in ensuring safety of a human body when power is supplied to the capsule endoscope inserted into the subject.

In view of the foregoing, an object of the present invention is to provide an energy supplying coil with high energy efficiency under low voltage, and a wireless system for obtaining information inside a subject using the energy supplying coil.

DISCLOSURE OF INVENTION

To solve the above problems and achieve the object, an energy supplying coil according to the present invention includes a coil that supplies energy to an introduction device used in a state of being inserted into a subject to execute a predetermined function within the subject. The coil is wound around an outside of the subject into which a body-insertable device passes, the body insertable device being disposed in an internal space of the wound coil.

In the energy supplying coil according to the present invention, a pitch of the coil is substantially constant.

In the energy supplying coil according to the present invention, the coil includes a plurality of loops, and a power feeder that connects power feeding points of the loops.

In the energy supplying coil according to the present invention, the coil includes a plurality of coils that form the internal space, each of the coils having more than one winding to which power is fed individually.

The energy supplying coil according to the present invention further includes a power feeding control unit feeding power to at least one of the coils based on a position of the body-insertable device.

In the energy supplying coil according to the present invention, a diameter of the coil is substantially the same as a diameter of the body of the subject.

In the energy supplying coil according to the present invention, a ratio of a radius of the coil forming the internal space to a length of the coil in an axial direction is 0.1 to 3.

In the energy supplying coil according to the present invention, a driving frequency of the coil is less than one megahertz.

In the energy supplying coil according to the present invention, a voltage supplied to the coil is less than 100 volts.

In the energy supplying coil according to the present invention, a self inductance of the coil is set so that a voltage obtained by multiplying a driving frequency of the coil, the self inductance of the coil, a current that flows through the coil, and $2\pi$ is less than 100 volts.

In the energy supplying coil according to the present invention, a resistance of the coil is less than 100 ohms.

In the energy supplying coil according to the present invention, the coil is embedded into or attached to a flexible material.

A wireless system for obtaining information inside a subject includes: an introduction device introduced into a subject; and a transmitting/receiving device disposed at an outside of the subject, acquiring information obtained by the introduction device via wireless communication, and supplying energy to the introduction device, wherein the introduction device includes: a function executing unit that executes a predetermined function; a wireless unit that wirelessly transmits information obtained by the function executing unit; and an energy receiving unit that receives the energy, and the transmitting/receiving device includes: a wireless receiving unit that receives information transmitted from the wireless unit; an energy supplying unit that includes an energy supplying coil according to any one of claims 1 to 12 and supplies energy to the energy receiving unit; and a processing unit that analyzes the received information.

BRIEF DESCRIPTION OF DRAWINGS

FIG. 1 is a schematic view showing a whole arrangement of a wireless system for obtaining information inside a subject according to a first embodiment of the present invention;
FIG. 2 is a schematic block diagram of a configuration - of a transmitting/receiving device that constitutes the wireless system for obtaining information inside the subject shown in Fig. 1;
FIG. 3 is a schematic block diagram of a configuration of a capsule endoscope that constitutes the wireless system for obtaining information inside the subject shown in Fig. 1;
Fig. 4 is a schematic configuration diagram of an energy supplying coil;
Fig. 5 depicts dependency of efficiency on a ratio of the coil length to the coil radius;
Fig. 6 depicts frequency dependence of an absorption coefficient of fresh water and seawater for electromagnetic waves;
Fig.7 depicts a magnetic field on an axis generated by a coil;
Fig. 8 is a schematic configuration diagram of an energy supplying coil according to a second embodiment of the present invention; and
Fig. 9 is a schematic configuration diagram of an energy supplying coil according to a third embodiment of the present invention.

BEST MODE(S) FOR CARRYING OUT THE INVENTION

Exemplary embodiments of the present invention will be explained in detail below with reference to the accompanying drawings. Note that the present invention is not limited to the specific embodiments.

First embodiment

A wireless system for obtaining information inside a subject according to a first embodiment will be explained. In the wireless system for obtaining information inside the subject according to the first embodiment, a capsule endoscope will be explained as one example of a body-insertable device.

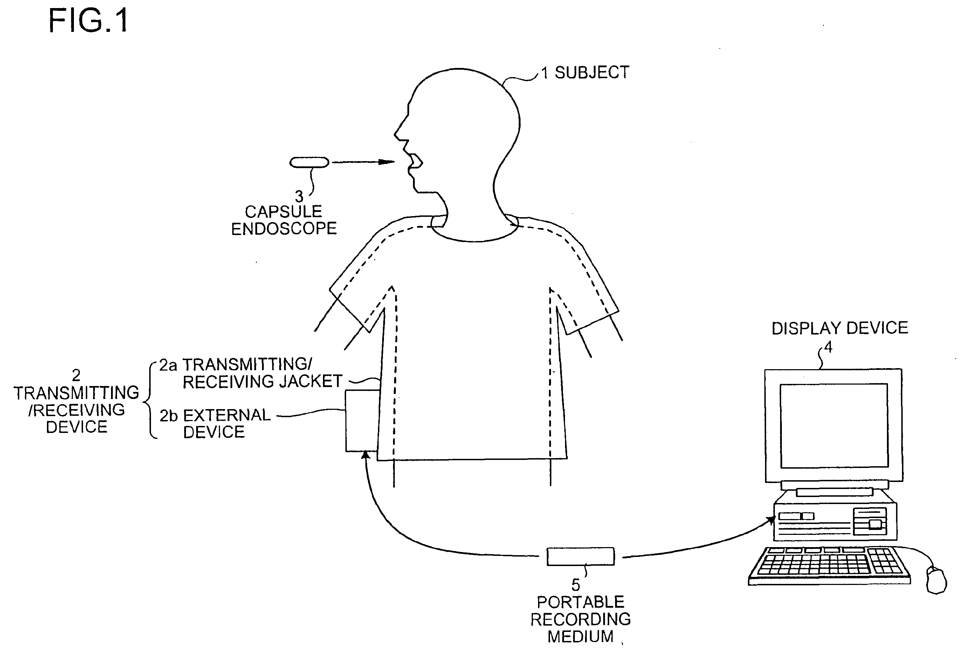

Fig. 1 is a schematic diagram of the wireless system for obtaining information inside the subject according to the first embodiment. As shown in Fig. 1, the wireless system for obtaining information inside the subject includes a transmitting/receiving device 2 having a wireless transmitting/receiving function, and a capsule endoscope 3 that is introduced into a subject 1, is operated by driving power obtained by a radio signal transmitted from the transmitting/receiving device 2, and captures images in a body cavity so as to transmit image data to the transmitting/receiving device 2. Further, the wireless system for obtaining information inside the subject includes a display device 4 that displays images in the body cavity based on the data received by the transmitting/receiving device 2, and a portable recording medium 5 that exchanges the data between the transmitting/receiving device 2 and the display device 4. The transmitting/receiving device 2 includes a transmitting/receiving jacket 2a worn by the subject 1, and an external device 2b that processes a radio signal transmitted and received via the transmitting/receiving jacket 2a.

The display device 4 is for displaying the images in the body cavity imaged by the capsule endoscope 3, and has a configuration of a work station or the like for displaying the images based on data obtained by the portable recording medium 5. Specifically, the display device 4 may have a configuration such that the images are displayed directly on a CRT display, a liquid crystal display or the like, or a configuration such that the images are output to another medium such as a printer.

The portable recording medium 5 is detachable from the external device 2b and the display device 4, and has a configuration such that when inserted to be attached to both of them, information can be output or recorded. Specifically, the portable recording medium 5 is inserted to be attached into the external device 2b so as to record data transmitted from the capsule endoscope 3 therein while the capsule endoscope 3 is moving in the body cavity of the subject 1. After the capsule endoscope 3 is ejected from the subject 1, namely, after the imaging of the inside of the subject 1 is completed, the portable recording medium 5 is taken out of the external device 2b and is inserted to be attached into the display device 4. The recorded data are read by the display device 4. The data are exchanged between the external device 2b and the display device 4 through the portable recording medium 5 such as a compact flash (registered trademark) memory. As a result, the subject 1 can move freely during the photographing of the body cavity unlike the case such that the external device 2b and the display device 4 are connected with each other by a wire.

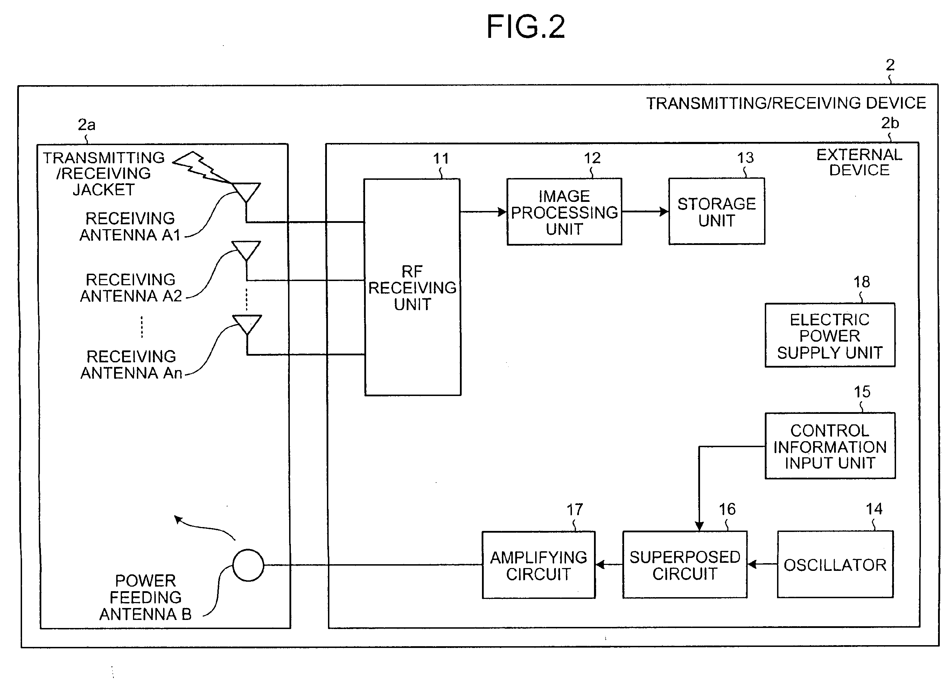

The transmitting/receiving device 2 has a function as a power feeding device for transmitting electric power to the capsule endoscope 3, and also a function as a receiving device that receives image data of the body cavity transmitted from the capsule endoscope 3. Fig. 2 is a schematic block diagram showing a configuration of the transmitting/receiving device 2. As shown in Fig. 2, the transmitting/receiving device 2 can be worn by the subject 1, and has the transmitting/receiving jacket 2a having receiving antennas A1 to An and a power feeding antenna B (an energy supplying coil), and the external device 2b that processes transmitted/received radio signals.

The external device 2b has a function for processing the radio signal transmitted form the capsule endoscope 3. Specifically, as shown in Fig. 2, the external device 2b includes an RF receiving unit 11 that executes a predetermined processing for a radio signal received by the receiving antennas A1 to An and extracts image data obtained by the capsule endoscope 3 from the radio signal so as to output the image data, an image processing unit 12 that executes a process necessary for the output image data, and a storage unit 13 that stores the image data executed the image process. The image data are recorded in the portable recording medium 5 via the storage unit 13.

The external device 2b has a function for generating a radio signal transmitted to the capsule endoscope 3. Specifically, the external device 2b includes an oscillator 14 that generates a power feeding signal and defines an oscillation frequency, a control information input unit 15 that generates a control information signal for controlling a driving state of the capsule endoscope 3, a superposed circuit 16 that synthesizes the power feeding signal with the control information signal, and an amplifying circuit 17 that amplifies strength of the synthesized signal. The signal amplified by the amplifying circuit 17 is transmitted to the power feeding antenna B so as to be transmitted to the capsule endoscope 3. The external device 2b includes an electric power supply unit 18 having a predetermined capacitor, an AC power source adapter or the like. The components of the external device 2b use electric power supplied from the electric power supply unit 18 as a driving energy.

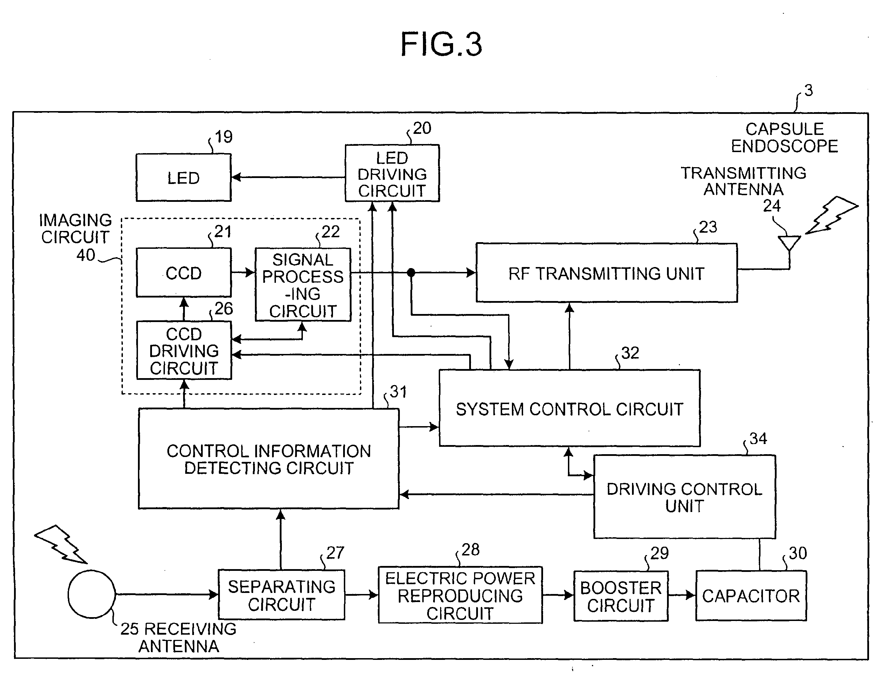

The capsule endoscope 3 will be explained. Fig. 3 is a schematic block diagram of a configuration of the capsule endoscope 3. As shown in Fig. 3, the capsule endoscope 3 includes a light emitting diode (LED) 19 that irradiates an imaging area when the inside of the subject 1 is captured, an LED driving circuit 20 that controls a driving state of the LED 19, a charge coupled device (CCD) 21 that images a reflected light image from the area irradiated by the LED 19, and a signal processing circuit 22 that processes an image signal output from the CCD 21 to obtain imaging information in a desired format. Further, the capsule endoscope 3 includes a CCD driving circuit 26 that controls a driving state of the CCD 21, an RF transmitting unit 23 that modulates the image data imaged by the CCD 21 and processed by the signal processing circuit 22 so as to generate an RF signal, a transmitting antenna 24 that transmits the RF signal output from the RF transmitting unit 23, and a system control circuit 32 that controls operations of the LED driving circuit 20, the CCD driving circuit 26, and the RF transmitting unit 23. The CCD 21, the signal processing circuit 22, and the CCD driving circuit 26 are collectively called an imaging circuit 40.

The capsule endoscope 3 acquires image information about portions to be examined irradiated by the LED 19 by the CCD 21 through these mechanisms while introduced into the subject 1. The signal processing circuit 22 executes the signal processing on the acquired image information, and after the RF transmitting unit 23 converts the image information into an RF signal, it transmits the RF signal to the outside via the transmitting antenna 24.

The capsule endoscope 3 includes a receiving antenna 25 that receives a radio signal transmitted from the transmitting/receiving device 2, and a separating circuit 27 that separates a power feeding signal from the signal received by the receiving antenna 25. Further, the capsule endoscope 3 includes an electric power reproducing circuit 28 that reproduces electric power from the separated power feeding signal, a booster circuit 29 that raises the reproduced electric power, and a capacitor 30 that stores the raised electric power. Further, the capsule endoscope 3 includes a control information detecting circuit 31 that detects contents of a control information signal from a component separated from the power feeding signal by the separating circuit 27, and outputs a control signal to the LED driving circuit 20, the CCD driving circuit 26, and the system control circuit 32 if necessary. The control information detecting circuit 31 and the system control circuit 32 also have a function for distributing the driving power supplied form the capacitor 30 to the other components.

The capsule endoscope 3 including these components receives a radio signal transmitted from the transmitting/receiving device 2 via the receiving antenna 25, and separates a power feeding signal and a control information signal from the received radio signal. The control information signal is output to the LED driving circuit 20, the CCD driving circuit 26, and the system control circuit 32 via the control information detecting circuit 31 so as to be used for controlling the driving states of the LED 19, the CCD 21, and the RF transmitting unit 23. On the other hand, the electric power reproducing circuit 28 reproduces the power feeding signal as electric power, and the booster circuit 29 raises the reproduced electric power up to a potential of the capacitor 30, so that the raised electric power is stored in the capacitor 30. The capacitor 30 has a function for being capable of supplying electric power to the system control circuit 32 and the other components. The capsule endoscope 3 is configured so that electric power is supplied by wireless transmission from the transmitting/receiving device 2.

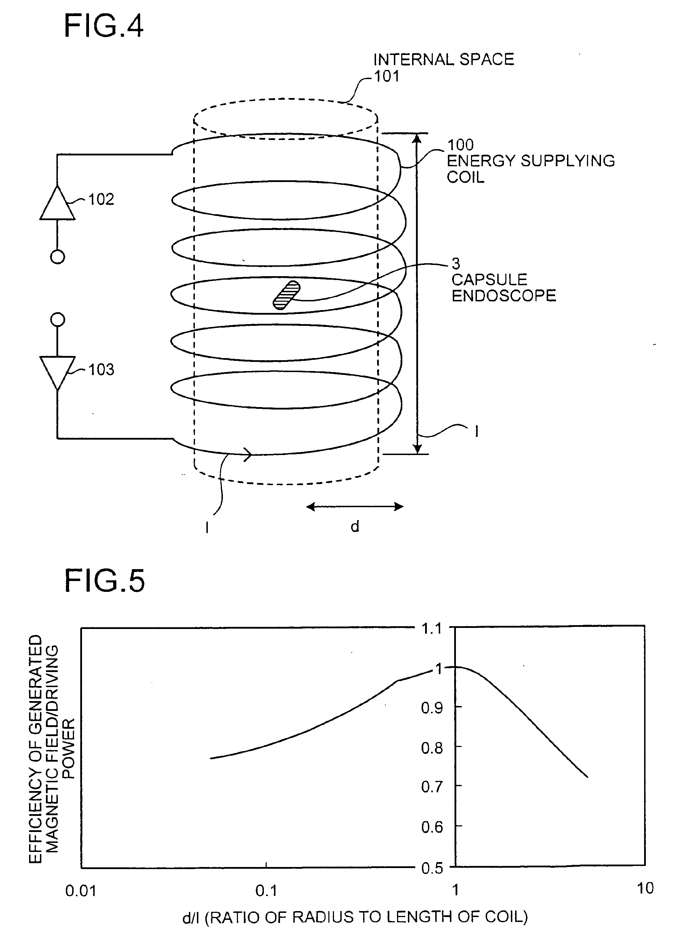

An energy supplying coil 100 as shown in Fig. 4 realizes the power feeding antenna B. As shown in Fig. 4, the energy supplying coil 100 is a conductor coiled around a cylindrical internal space 101 into which the subject 1 is positioned. The energy supplying coil 100 supplies power to a capsule endoscope 3 based on a magnetic coupling power supply. The energy supplying coil 100 is a cylindrical solenoid coil loosely wound to have a low self inductance. Amplifiers 102 and 103 are provided at both ends of the energy supplying coil 100, and correspond to the amplifier circuit 17 of the external device 2b shown in Fig. 2. The energy supplying coil 100 is embedded into or is attached to the transmitting/receiving jacket 2a. The transmitting/receiving jacket 2a is formed with a flexible material. The energy supplying coil 100 has a large length to form the internal space for providing a certain room around the body of the subject 1. The transmitting/receiving jacket 2a having a stretching property fits well with the shoulders and the body of the subject 1, and the energy supplying coil 100 is closely contacted to the subject 1. The energy supplying coil 100 forms the internal space 101 at a position corresponding to a position where the capsule endoscope 3 passes through the subject 1. The pitch of the energy supplying coil 100 is set at substantially the same level.

A magnetic field H at the center of the energy supplying coil 100 is expressed as

$$H = N \cdot I / \sqrt{(l^2 + d^2)}$$

where l is a length of the energy supplying coil 100 in the axial direction, d is a radius, N is the number of turns, and I is a current that flows therethrough. The magnetic field H at the center of the coil is proportional to the number of turns N and the current I. On the other hand, a self inductance L of the energy supplying coil 100 is expressed as

$$L = K \cdot \mu \cdot N^2 \cdot \pi \cdot d^2 / l$$

where K is a Nagaoka coefficient, and $\mu$ is a permeability.
A counter electromotive force Vr when the current I flows through the energy supplying coil 100 is approximated to

$$Vr = 2\pi \cdot f \cdot L \cdot I.$$

Therefore, a voltage V that is necessary to drive the energy supplying coil 100 is approximated to

$$V = 2\pi \cdot f \cdot L \cdot I$$

where f is a frequency of an alternating current supplied to the energy supplying coil 100.
Therefore, efficiency $\eta$ of the generated magnetic field H to driving force (V.I) is expressed as

$$\eta = H / (V \cdot I)$$
$$= N \cdot I / \sqrt{(l^2 + d^2)} / (2\pi \cdot f \cdot L \cdot l^2).$$

When the efficiency $\eta$ where the number of turns N, the radius d, the frequency f, and the current I are constant is expressed as efficiency q, the efficiency $\zeta$ is expressed as

$$\zeta = 1 / (K2 \cdot \sqrt{(1 + (d/l)^2)})$$

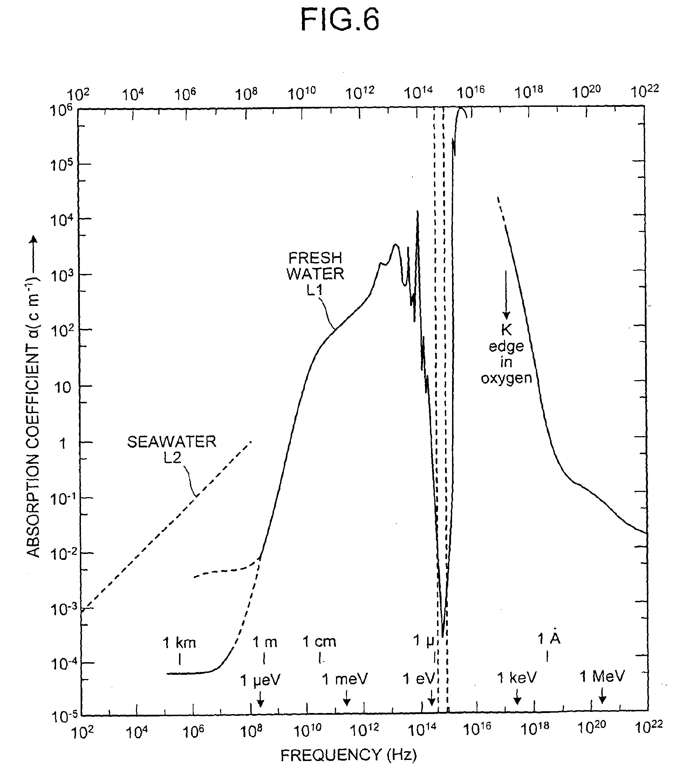

where K2 is a proportionality constant. Energy transmission efficiency can be maximized when the energy supplying coil 100 has the length l that maximizes this efficiency q.
The dependency of the efficiency $\zeta$ on a ratio of the radius d to the length l, (d/l), is obtained as shown in Fig. 5. As shown in Fig. 5, the efficiency $\zeta$ becomes maximum when the ratio (d/l) is 1, and the efficiency $\zeta$ of 80 percent or above is obtained when the ratio (d/l) is within a range from 0.1 to 3.
The frequency f of the current I applied to the energy supplying coil 100 will be explained next. Fig. 6 depicts frequency dependence of an absorption coefficient of fresh water and seawater for electromagnetic waves. In Fig. 6, a curve L1 in a solid line is an absorption coefficient of fresh water, and a curve L2 in a broken line is an absorption coefficient of seawater. The structure of a human body can be approximated to seawater close to physiological saline, and, therefore, an absorption coefficient of the human body can be taken as the absorption coefficient of seawater. The absorption coefficient of seawater becomes large along an increase in the frequency, and the absorption coefficient becomes substantially one when the frequency is $10^8$ hertz. The absorption coefficient one means that energy is substantially absorbed in the length of 1 centimeter. In other words, when the frequency is high, such as about gigahertz, substantially all the quantity of energy supplied is absorbed in the human body, and the energy does not reach the capsule endoscope 3. When the human body is considered, the frequency f not more than one megahertz at which the absorption length becomes 10 centimeters or above needs to be selected. In actual practice, when the human body has a height of about 100 centimeters, it is suitable to select a frequency band of 100 kilohertz. In this case, the absorption coefficient is about $10^{-2}$.

The voltage V to be applied to the energy supplying coil 100 is preferably about 100 volts or less. The magnetic field to be applied from the energy supplying coil 100 to the subject 1 needs to fulfill the laws. As the self inductance is $L=K\cdot\mu\cdot N^2\cdot n\cdot d^2/l$, the voltage V must fulfill the following

$$V=2\pi\cdot f\cdot L\cdot I$$
$$=2\pi\cdot f\cdot I\cdot K\cdot\mu\cdot N^2\cdot\pi\cdot d^2/l$$
$$\leqq 100\ (V).$$

When the magnetic field at the center of the coil under the laws is Hc, the magnetic field H at the center of the coil must fulfill the following

$$H=N\cdot I/\sqrt{(l^2+d^2)}$$
$$< Hc.$$

The above V and H limit the current I and the number of turns N.

As the loss of energy due to Joule heat affects the efficiency, a resistance R of the energy supplying coil 100 is preferably less than 100 ohms.

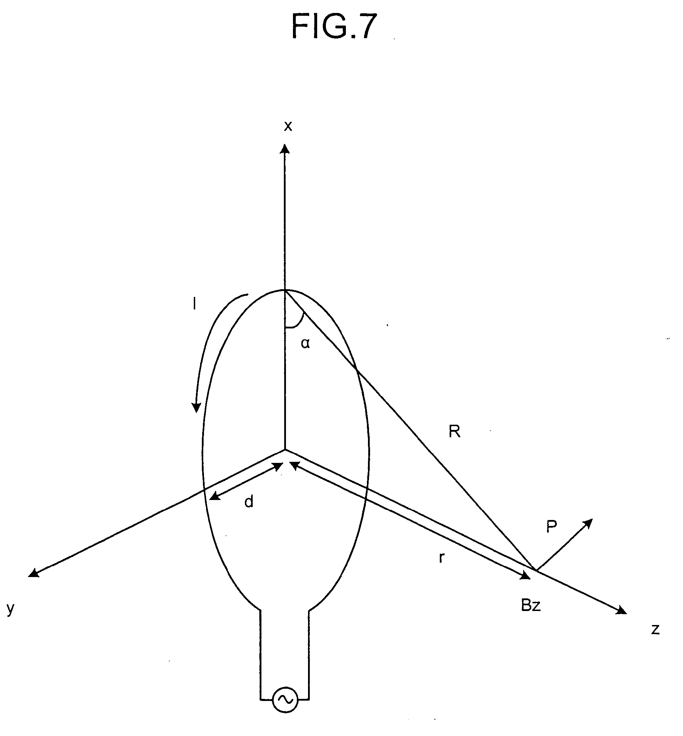

A lower limit and an upper limit of the number of turns N of the energy supplying coil 100 will be explained. The lower limit of the number of turns N will be explained first. When a single coil is disposed as shown in Fig. 7, a magnetic field Bz on the coil center axis (z axis) is expressed as

$$Bz=\mu\cdot I\cdot N\cdot d^2/(2(d^2+r^2)^{3/2})$$

where r is a distance from the coil center. When the value of $\mu.I.N/2$ as a constant is K3, and r=0 (meter), the following expression is obtained:

$$Bz=K3\cdot d^2/((d^2+r^2)^{3/2})$$
$$=K3/d.$$

A ratio LB of the reduction of the magnetic field Bz at a constant distance r is expressed as

$$LB= K3\cdot d^2/((d^2+r^2)^{3/2})/(K3/d)$$
$$= d^3/((d^2+r^2)^{3/2}).$$

When the magnetic field Bz is interpolated by a magnetic field of an opposite coil r at a position where the magnetic field Bz is lowered to 50 percent on the z axis, the magnetic field which is lowered to 50 percent can be the same as that at the position where r=0 (meter). This r is obtained as r=0.766.d from the following expression:

$$0.5= d^3/((d^2+r^2)^{3/2}).$$

When d is equal to 0.15 meter, r is equal to 0.115 meter or 11.5 centimeters. Because the distance r is a half of the coil distance (pitch), a maximum coil distance becomes 23 centimeters. When the body length is 40 centimeters or above, the coil needs to be wound around the body by at least three turns. When margin is allowed by taking a bend of the body into account, the coil needs to be wound by at least four turns.

The upper limit of the number of turns N of the energy supplying coil 100 will be explained next. The upper limit of the number of turns N can be obtained from inductive reactance of the coil. Inductive reactance X1 having a dimension of resistance can be expressed as $X1=2\pi\cdot f\cdot L$. Because the self inductance L of the energy supplying coil 100 is $L=K\cdot\mu\cdot N^2\cdot\Pi\cdot d^2/l$, the inductive reactance Xl can be expressed as

$$X1=2\pi\cdot f\cdot K\cdot\mu\cdot N^2\cdot\pi\cdot d^2/l.$$

When the inductive reactance which is about the same as the resistance R of the energy supplying coil 100 occurs, the inductance of the coil becomes a main cause of power loss. When the upper limit of the resistance R is 100 ohms,

$$R=100\ ohms$$
$$=X1$$
$$=2\pi\cdot f\cdot K\cdot\mu\cdot N^2\cdot\pi\cdot d^2/l.$$

N is equal to 29, when f=100 kilohertz, K=0.85, $\mu=4\Pi\cdot10^{-7}$, d=0.15 meter, and 1=0.4 meter. In other words, when the frequency is 100 kilohertz, the coil needs to be wound by not more than 29 turns.

In comparing the power efficiency of the counter coil that forms a magnetic field using a counter coil with the power efficiency of the cylindrical solenoid coil according to the first embodiment, the cylindrical solenoid coil generates the magnetic field five times as strong as the counter coil. It is subject to a condition that the same current flows through the coil having the same resistance.

According to the first embodiment, because the self inductance of the energy supplying coil 100 is set low, the driving voltage when the same alternating current flows through the coil can be restricted to a low level. Therefore, the occurrence of Joule heat due to the series parasitic resistance of the coil can be decreased to restrict reactive power, thereby enhancing the energy transmission efficiency. Further, the low setting of the self inductance of the energy supplying coil 100 avoids the occurrence of high voltage across the terminals of the coil. As a result, safety of the human body can be ensured. Second embodiment

A second embodiment of the present invention will be explained next. While the cylindrical solenoid coil is used for the energy supplying coil according to the first embodiment, a plurality of loops are connected to a common power feeder according to the second embodiment.

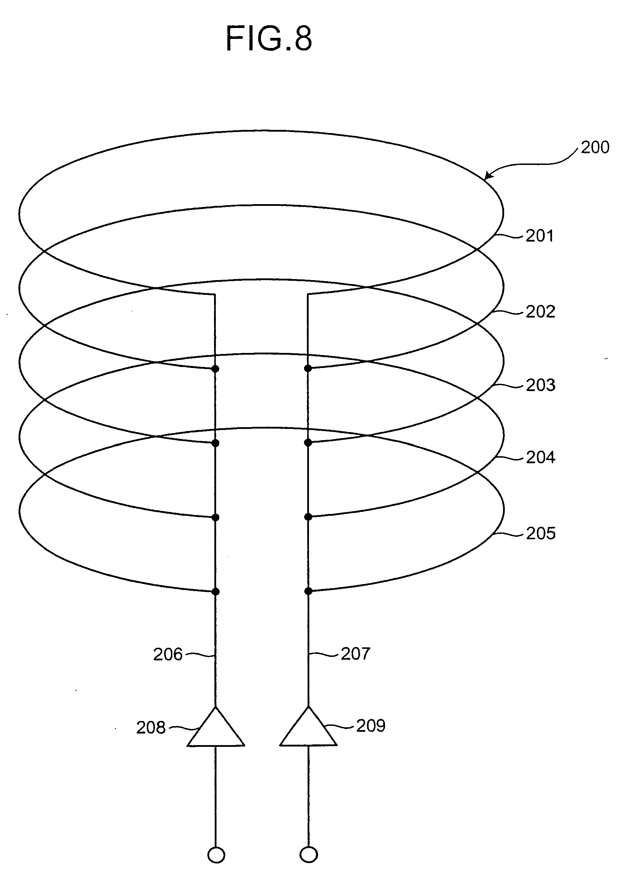

Fig. 8 is a schematic configuration diagram of the energy supplying coil according to the second embodiment of the present invention. As shown in Fig. 8, an energy supplying coil 200 is configured to connect a plurality of loops 201 to 205 to power feeders 206 and 207 at respective ends.

According to the second embodiment, a magnetic field similar to that according to the first embodiment can be formed, and the power feeders 206 and 207 can develop the loops 201 to 205. In other words, when the power feeders 206 and 207 are provided at the front of the transmitting/receiving jacket 2a, the jacket can be opened between the power feeders 206 and 207. This arrangement can avoid the need of an electrical connection between the coils.

To make the current flowing in each of the loops 201 to 205 the same in value, the resistances of the power feeders 206 and 207 are set to be substantially lower than those of the loops 201 to 205.

Third embodiment

A third embodiment according to the present invention will be explained next. According to the third embodiment, a plurality of loops form the energy supplying coil, and individually feed power to each loop.

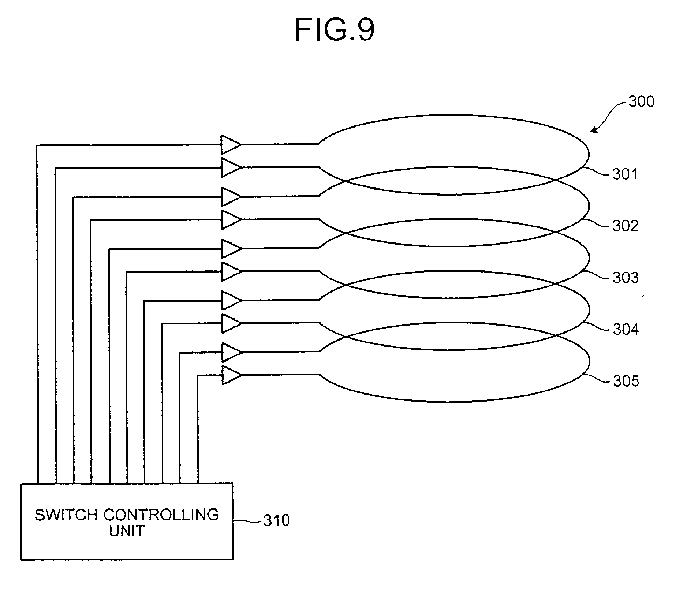

Fig. 9 is a schematic configuration diagram of the energy supplying coil according to the third embodiment of the present invention. In Fig. 9, an energy supplying coil 300 has a plurality of loops 301 to 305, which individually feed power. Power feeders of the loops 301 to 305 are connected to a switch controlling unit 310. The switch controlling unit 310 controls to feed power to at least one loop corresponding to the position of the capsule endoscope 3 in the internal space formed by the loops 301 to 305. The switch controlling unit 310 is provided within the external device 2b, and switch controls based on the position information of the capsule endoscope 3.

In the third embodiment, because power is supplied to only minimum necessary loops, power can be consumed efficiently.

The energy supplying coil according to the present invention has a coil shape or has coil arrangement to minimize the influence of the self inductance. Therefore, the efficiency of energy supply to a body-insertable device can be improved remarkably. Further, because the supply voltage can be minimized, it is advantageous that the safety of the human body can be ensured.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

INDUSTRIAL APPLICABILITY

In view of the foregoing, the present invention is suitable for an energy supplying coil that supplies energy from the outside of a subject to a body-insertable device used in a state of being inserted into the subject to execute a predetermined function within the subject, and a wireless system for obtaining information inside the subject using the energy supplying coil.

**Claims**

1.  An energy supplying coil for supplying energy to a body-insertable device used in a state of being inserted into a subject to execute a predetermined function within the subject, the energy supplying coil being wound around an outside of the subject into which the body-insertable device passes, the body-insertable device being disposed in an internal space of the would coil.

2.  The energy supplying coil according to claim 1, wherein a pitch of the coil is substantially constant.

3.  The energy supplying coil according to claim 1, wherein the coil comprises a plurality of loops; and a power feeder that connects power feeding points of the loops.

4.  The energy supplying coil according to claim 1, wherein the coil includes a plurality of coils that form the internal space, each of the coils having more than one winding to which power is fed individually.

5.  The energy supplying coil according to claim 4, further comprising a power feeding control unit that feeds power to at least one of the coils based on a position of the body-insertable device.

6. The energy supplying coil according to claim 1, wherein a diameter of the coil is substantially the same as a diameter of the body of the subject.

7. The energy supplying coil according to claim 1, wherein a ratio of a radius of the coil forming the internal space to a length of the coil in an axial direction is 0.1 to 3.

8. The energy supplying coil according to claim 1, wherein a driving frequency of the coil is less than one megahertz.

9. The energy supplying coil according to claim 1, wherein a voltage supplied to the coil is less than 100 volts.

10. The energy supplying coil according to claim 1, wherein a self inductance of the coil is set so that a voltage obtained by multiplying a driving frequency of the coil, the self inductance of the coil, a current flowing through the coil, and $2\pi$ is less than 100 volts.

11. The energy supplying coil according to claim 1, wherein a resistance of the coil is less than 100 ohms.

12. The energy supplying coil according to claim 1, wherein the coil is embedded into or attached to a flexible material.

13. A wireless system for obtaining information inside a subject comprising
a body-insertable device inserted into a subject; and
a transmitting/receiving device disposed at an outside of the subject, acquiring information obtained by the body-insertable device via wireless communication, and supplying energy to the body-insertable device, wherein
the body-insertable device includes
a function executing unit that executes a predetermined function;
a wireless unit that wirelessly transmits information obtained by the function executing unit; and
an energy receiving unit that receives the energy, and
the transmitting/receiving device includes
a wireless receiving unit that receives information transmitted from the wireless unit);
an energy supplying unit that includes an energy supplying coil wound around an outside of the subject into which the body-insertable device passes, the body-insertable device being disposed in an internal space of
the coil, and supplies energy to the energy receiving unit; and
a processing unit that analyzes the received information.

14. The wireless system for obtaining information inside the subject according to claim 13, wherein a pitch of the coil is substantially constant.

15. The wireless system for obtaining information inside the subject according to claim 13, wherein the coil comprises a plurality of loops, and a power feeder that connects power feeding points of the loops.

16. The wireless system for obtaining information inside the subject according to claim 13, wherein the coil includes a plurality of coils that form the internal space, each of the coils having more than one winding to which power is fed individually.

17. The wireless system for obtaining information inside the subject according to claim 16, further comprising a power feeding control unit that feeds power to at least one of the coils based on a position of the body-insertable device.

18. The wireless system for obtaining information inside the subject according to claim 13, wherein a diameter of the coil is substantially the same as a diameter of the body of the subject.

19. The wireless system for obtaining information inside the subject according to claim 13, wherein a ratio of a radius of the coil forming the internal space to a length of the coil in an axial direction is 0.1 to 3.

20. The wireless system for obtaining information inside the subject according to claim 13, wherein a driving frequency of the coil is less than one megahertz.

21. The wireless system for obtaining information inside the subject according to claim 13, wherein a voltage supplied to the coil is less than 100 volts.

22. The wireless system for obtaining information inside the subject according to claim 13, wherein a self inductance of the coil is set so that a voltage obtained by multiplying a driving frequency of the coil, the self inductance of the coil, a current that flows through the coil, and $2\pi$ is less than 100 volts.

23. The wireless system for obtaining information inside' the subject according to claim 13, wherein a resistance of the coil is less than 100 ohms.

24. The wireless system for obtaining information inside the subject according to claim 13, wherein the coil is embedded into or adhered to a flexible material.

# FIG.1

1 SUBJECT

3
CAPSULE
ENDOSCOPE

2
TRANSMITTING
/RECEIVING
DEVICE
{
2a TRANSMITTING/
RECEIVING JACKET

2b EXTERNAL
DEVICE
}

DISPLAY DEVICE
4

5
PORTABLE
RECORDING
MEDIUM

EP 1 665 977 A1

# FIG.2

TRANSMITTING/RECEIVING DEVICE    2

2a    2b

TRANSMITTING /RECEIVING JACKET

EXTERNAL DEVICE

RECEIVING ANTENNA A1

RECEIVING ANTENNA A2

RECEIVING ANTENNA An

11   RF RECEIVING UNIT

12   IMAGE PROCESSING UNIT

13   STORAGE UNIT

18   ELECTRIC POWER SUPPLY UNIT

15   CONTROL INFORMATION INPUT UNIT

17   AMPLIFYING CIRCUIT

16   SUPERPOSED CIRCUIT

14   OSCILLATOR

POWER FEEDING ANTENNA B

EP 1 665 977 A1

# FIG.3

CAPSULE ENDOSCOPE 3

19 LED

20 LED DRIVING CIRCUIT

IMAGING CIRCUIT 40

21 CCD

22 SIGNAL PROCESS-ING CIRCUIT

26 CCD DRIVING CIRCUIT

TRANSMITTING ANTENNA 24

23 RF TRANSMITTING UNIT

31 CONTROL INFORMATION DETECTING CIRCUIT

32 SYSTEM CONTROL CIRCUIT

34 DRIVING CONTROL UNIT

25 RECEIVING ANTENNA

27 SEPARATING CIRCUIT

28 ELECTRIC POWER REPRODUCING CIRCUIT

29 BOOSTER CIRCUIT

30 CAPACITOR

EP 1 665 977 A1

## FIG.4

INTERNAL SPACE
101

102

103

100
ENERGY SUPPLYING
COIL

3
CAPSULE
ENDOSCOPE

l

l

d

## FIG.5

EFFICIENCY OF GENERATED MAGNETIC FIELD/DRIVING POWER

1.1

1

0.9

0.8

0.7

0.6

0.5

0.01            0.1            1            10

d/l (RATIO OF RADIUS TO LENGTH OF COIL)

# FIG.6

# FIG.7

# FIG.8

# FIG.9

EP 1 665 977 A1

**INTERNATIONAL SEARCH REPORT**

| International application No. |
|---|
| PCT/JP2004/013294 |

A. CLASSIFICATION OF SUBJECT MATTER
Int.Cl$^7$ A61B1/00

According to International Patent Classification (IPC) or to both national classification and IPC

B. FIELDS SEARCHED

Minimum documentation searched (classification system followed by classification symbols)
Int.Cl$^7$ A61B1/00-1/32

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br><br>A<br><br>Y | WO 02/080753 A2 (GIVEN IMAGING LTD.),<br>17 October, 2002 (17.10.02),<br>Full text; Figs. 1 to 4<br><br>Full text; Figs. 1 to 4<br>(Family: none)<br><br>Microfilm of the specification and drawings<br>annexed to the request of Japanese Utility<br>Model Application No. 187419/1987(Laid-open<br>No. 93930/1989)<br>21 June, 1989 (21.06.89),<br>Full text; Fig. 1<br>(Family: none) | 1,2,4,6,13,<br>14,16,18<br>3,5,7-12,15,<br>17,19-24<br><br>1,2,4,6,13,<br>14,16,18 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 September, 2004 (22.09.04) | Date of mailing of the international search report<br>12 October, 2004 (12.10.04) |
|---|---|
| Name and mailing address of the ISA/<br>Japanese Patent Office | Authorized officer |
| Facsimile No. | Telephone No. |

Form PCT/ISA/210 (second sheet) (January 2004)

17

**INTERNATIONAL SEARCH REPORT**

| International application No. |
| --- |
| PCT/JP2004/013294 |

C (Continuation). DOCUMENTS CONSIDERED TO BE RELEVANT

| Category* | Citation of document, with indication, where appropriate, of the relevant passages | Relevant to claim No. |
| --- | --- | --- |
| E,X<br><br>E,A | JP 2004-159456 A  (Kabushiki Kaisha Aiden Bideotoronikusu),<br>03 June, 2004 (03.06.04),<br>Full text; Figs. 1 to 11<br><br>Full text; Figs. 1 to 11<br>(Family: none) | 1-4,6,13-16, 18<br>5,7-12,17, 19-24 |

Form PCT/ISA/210 (continuation of second sheet) (January 2004)